# EUROPEAN PATENT APPLICATION

(11) **EP 1 365 242 A1**
(43) Date of publication of application: **26.11.2003**
(21) Application number: 02010276.0
(22) Date of filing: 21.05.2002
(51) Int. Cl.: G01N 33/574, C12Q 1/68, C07K 14/47, A61P 35/00

(54) **Compounds for detection and treatment of colorectal lesions**

(71) Applicant: MTM Laboratories AG, 69120 Heidelberg (DE)
(72) Inventor: Hipfel, Rainer, 69126 Heidelberg (DE); Coy, Johannes, 63762 Grossostheim (DE)

(57) **Abstract**

The present invention relates to molecules associated with colorectal lesions. The invention is more specifically related to polypeptides and nucleic acids comprising at least portions of nucleic acids or polypeptides, the expression of which is significantly altered in association with colorectal lesions. Such combinations of nucleic acids and/or polypeptides may be used for enhanced diagnosis, progression monitoring and assessment of prognosis of colorectal lesions. Furthermore the present invention provides a method for early diagnosis, assessment of prognosis and monitoring of the disease course and for therapy and vaccination of colorectal lesions.

## Description

The present invention relates to molecules associated with colorectal lesions. The invention is more specifically related to polypeptides and nucleic acids comprising at least portions of nucleic acids or polypeptides, the expression of which is significantly altered in association with colorectal lesions. The nucleic acids and/or polypeptides of the present invention may be used for enhanced diagnosis, progression monitoring and assessment of prognosis of colorectal lesions. Furthermore the present invention provides a method for early diagnosis, assessment of prognosis and monitoring of the disease course and for therapy of colorectal lesions and vaccination of individuals against said lesions.

In most tumors there is a strong correlation between the patients outcome following initial therapy and the stage at which the disease has been diagnosed. So the earlier the cancer could be detected the better are the chances for the patient to survive. Thus sensitive testing methods are required for detecting the tumors in early stages.

The most promising methods for early diagnosis of tumors are those involving molecular markers characteristic for tumor cells.

Cancer is a quite heterogeneous disease. Multiple regulators of the cell growth can be involved in the genesis of cancer. These regulatory elements of the cell cycle can be either positive regulators, named oncogenes when mutated, so that a transformed state is reached, or negative regulators, named tumor suppressor genes. The number of factors known to be involved in the regulation of the cell cycle and potentially being candidates for the development of cancer exceeds 100 up to now and is still increasing.

The molecules being involved in the emergence of the cancerous state of a cell can be used to discriminate between cancer cells and normal tissue. Thus cancerous tissue can be detected by detecting molecules characteristic for the cancer cells. This turns out to be sophisticated due to the large number of molecules potentially being involved in causing cancer.

There are several molecular markers being characteristic of the gross number of cancers. But still there remains a certain percentage of tumors, that could not be detected using those markers.

For improved diagnosis of tumors there is a need for new marker molecules for use in diagnosis.

The present invention provides compounds such as nucleic acids and polypeptides associated with colorectal cancer. According to the present invention theses sequences may be used as marker molecules that allow for comprehensive detection of colorectal lesions even at early stages.

The present invention thus provides polypeptides and nucleic acids comprising at least portions of nucleic acids or polypeptides, the expression of which is significantly altered associated with colorectal lesions, that allow enhanced prognosis and diagnosis of diseases associated with abnormalities of the growth of cells.

In another aspect of the invention nucleic acids and/or polypeptides or combinations thereof may be used for therapy and/or vaccination of diseases associated with disorders of cell growth.

Yet another aspect of the present invention are pharmaceutical compositions containing polypeptides and/or polynucleotides disclosed herein or combinations thereof optionally together with one or more other therapeutic or diagnostic agents and/or carrier or adjuvant substances.

The present invention also provides kits such as diagnostic kits or research kits comprising the polynucleotides or polypeptides disclosed herein or combinations thereof.

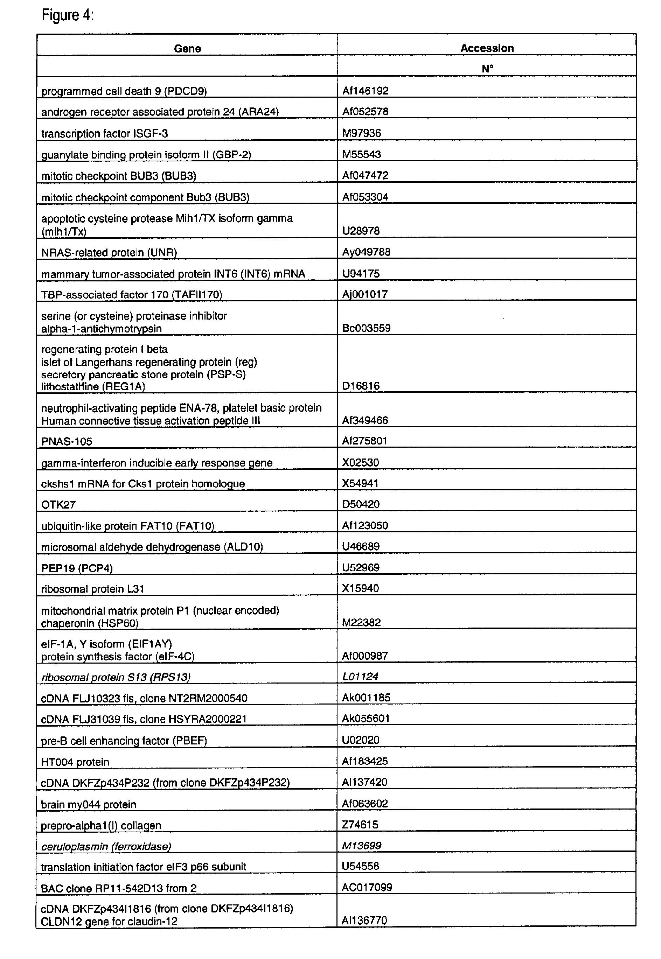

The present invention is based on the inventors findings shown in the examples 1- 2, that the level of expression of nucleic acids or polypeptides of the genes given in Figure 4 or fragments thereof in samples allows to diagnose, stage and grade diseases, to predict the course of the disease and to follow up the disease after initial therapy.

In one aspect the method according to the present invention is especially useful for early detection of disorders and for detection of disseminated tumor cells in the course of diagnosis of minimal residual disease.

In another aspect the present invention may be used for the diagnosis of tumors in samples such as tumor resections, biopsies or the like. In this aspect the invention provides a method, that allows to build a strategy for the therapy of diseases according to their molecular properties. According to the present invention the level of said polypeptides or nucleic acids can be used as a molecular marker for assessment of prognosis, monitoring and the design of a strategy of tumor therapeutics.

The present invention provides and makes use of compounds such as nucleic acids or polypeptides, the expression of which is significantly altered in association with colorectal lesions.

An alteration is said to be in association with colorectal lesions as used in the context of the present invention, if said alteration in comparison to control tissue occurs in cells and/or tissues affected by said lesion. An expression pattern (overexpression or loss of expression or expression of altered sequences) of nucleic acids and polypeptides is associated with colorectal lesions as used herein, if the expression pattern is detectable in colorectal lesions and is not or not significantly detectable in normal control tissue. A nucleic acid or polypeptides is associated with colorectal lesions as used herein, if the expression of the nucleic acid or polypeptide is altered in association with colorectal lesions.

The expression of a compound is said to be significantly altered according to the present invention, if the level of expression differs by more than 30%. The alteration of the expression may comprise for example elevated expression or reduced expression of said compound. Another aspect of the altered expression may be an alteration in a way, that the compound is expressed under non wild-type circumstances. This may comprise, that the compound is for example expressed in situations, that naturally suppress the expression, or is not expressed in situations, that naturally induce the expression of the compound. Expression as used according to the present invention may comprise for example expression of proteins. The transcription to RNA and thus the level of mRNA may also be understood to be expression according to the present invention. The alteration of the expression is said to be in association with a colorectal lesion if it occurs in a representative number of cases afflicted with said disease compared to non affected test samples.

Nucleic acids as used in the context of the present invention are preferably polynucleotides or fragments thereof. Preferred polynucleotides comprise at least 20 consecutive nucleotides, preferably at least 30 consecutive nucleotides and more preferably at least 45 consecutive nucleotides, that are identical, share sequence homology or encode for identical, or homologous polypeptides, compared to the polypeptides associated with the proliferative disorders disclosed herein. The nucleic acids according to the present invention may also be complementary to any of said polynucleotides. Polynucleotides may for example include single-stranded (sense or antisense) or double-stranded molecules, and may be DNA (genomic, cDNA or synthetic) or RNA. RNA molecules comprise as well hnRNA (containing introns) as mRNA (not containing introns). According to the present invention the polynucleotides may also be linked to any other molecules, such as support materials or detection marker molecules, and may, but need not, contain additional coding or non-coding sequences.

The polynucleotides according to the present invention may be native sequences or variants thereof. The variants may contain one or more substitutions, additions, deletions and/or insertions such that the immunogenicity of the encoded polypeptide is not diminished, relative to the respective native proteins. The variants show preferably 70%, more preferably at least 80% and most preferably at least 90% of sequence identity to the native nucleic acid molecules used in the methods according to the present invention. Methods for determination of sequence similarity are known to those of skill in the art.

One example for detecting the similarity of sequences can be carried out using the FastA and/or BlastN bioinformatics software accessible on the HUSAR server of the DKFZ Heidelberg.

Furthermore nucleic acids useful for performing the method of the present invention are all polynucleotides, which hybridise to probes specific for the sequences disclosed herein under stringent conditions. Stringent conditions applied for the hybridisation reaction are known to those of ordinary skill in the art and may be applied as described in Sambrook et al. Molecular cloning: A Laboratory Manual, 2^{nd} Edition, 1989.

The present invention also makes use of polynucleotides, that due to the degeneracy of the genetic code encode the polypeptides natively encoded by the disclosed nucleic acids while not showing the percentage of sequence homology as described above within the nucleic acid sequence. Such nucleic acids might arise by changing the codons present in the disclosed sequences by degenerate codons and so preparing a synthetic nucleic acid.

The nucleotide sequences according to the present invention may be joined to a variety of other nucleic acid sequences using the known recombinant DNA techniques. The sequences may for example be cloned into any of a variety of cloning vectors, such as plasmids, phagemids, lambda phage derivatives and cosmids. Furthermore vectors such as expression vectors, replication vectors, probe generation vectors and sequencing vectors may be joined with the sequences disclosed herein. Sequences of special interest, that could be cloned to the nucleic acids according to the present invention are for example non coding sequences and regulatory sequences including promoters, enhancers and terminators.

In a preferred embodiment polynucleotides may be formulated such, that they are able to enter mammalian cells and to be expressed in said cells. Such formulations are especially useful for therapeutic purposes. The expression of nucleic acid sequences in target cells may be achieved by any method known to those skilled in the art. The nucleic acids may for example be joined to elements that are apt to enable their expression in a host cell. Such elements may comprise promoters or enhancers, such as CMV-, SV40-, RSV-, metallothionein I- or polyhedrin-promotors respectively CMV- or SV40-enhancers. Possible methods for the expression are for example incorporation of the polynucleotides into a viral vector including adenovirus, adeno-associated virus, retrovirus, vaccinia virus or pox virus. Viral vectors for the purpose of expression of nucleic acids in mammalian host cells may comprise pcDNA3, pMSX, pKCR, pEFBOS, cDM8, pCEV4 etc.. These techniques are known to those skilled in the art.

Other formulations for administration in therapeutic purposes include colloidal dispersion systems such as for example macromolecule complexes, microspheres, beads, micelles and liposomes.

Polypeptides as used in the present invention comprise at least an immunogenic portion of the proteins, that show significant alterations in their expression associated with colorectal lesions. The polypeptides may be of any length. Immunogenic portion as used above is a portion of a protein, that is recognized by a B-cell and/or T-cell surface antigen receptor. The immunogenic portions comprise at least 10 amino acid residues, more preferably at least 20 amino acid residues of the protein associated with a colorectal lesion. In a preferred embodiment of the present invention, particular domains of the proteins, such as for example transmembrane domains or N-terminal leader sequences have been deleted. The immunogenic portions according to the present invention react with antisera or specific antibodies in the same or nearly same intensity as the native full length proteins.

The immunogenic portions are generally identified using the techniques well known in the art. Possible techniques are for example screening of the polypeptides for the ability to react with antigen-specific antibodies, antisera and/or T-cell lines or clones.

The polypeptides associated with proliferative disorders used in combinations according to the present invention comprise also variants of the native proteins. These variants may differ from the native protein in one or more alterations such as substitutions, deletions, additions and/or insertions. The immunoreactivity of the variants according to the present invention is not substantially diminished compared to the native proteins. In a preferred embodiment of the invention the immunoreactivity is diminished less than 50% in a more preferred embodiment the immunoreactivity is diminished less than 20 % compared to the native polypeptides.

In a preferred embodiment variants may be deficient in one or more portions, such as for example N-terminal leader sequences, transmembrane domains or small N- and/or C-terminal sequences. The variants exhibit 70%, more preferably at least 90% and most preferably at least 95% identity to the polypeptides disclosed according to the present invention.

The variants used according to the present invention are preferably conservative substitutions, so that the amino acids changed are substituted for amino acids with similar properties. The properties concerned may include polarity, charge, solubility, hydrophobicity, hydrophilicity and/or amphipathic nature of the amino acid residues.

The variants useful for performing the methods disclosed herein may also comprise additional terminal leader sequences, linkers or sequences, which enable synthesis, purification or stability of the polypeptides in an easier or more comfortable way.

The polypeptides may comprise fusion or chimeric polypeptides containing sequences disclosed herein.

The polypeptides and polynucleotides according to the present invention are isolated. This means that the molecules are removed from their original environment. Naturally occurring proteins are isolated if they are separated from some or all of the materials, which coexist in the natural environment. Polynucleotides are isolated for example if they are cloned into vectors.

Furthermore the present invention makes use of agents such as antibodies and antigen-binding fragments, that specifically bind to the proteins associated with a colorectal lesion. The antibody or antigen-binding agent is said to react specifically, if it reacts at a detectable level with a protein disclosed herein, and does not significantly react with other proteins. The antibodies according to the present invention may be monoclonal or polyclonal antibodies. Other molecules capable of binding specifically may be for example antigen-binding fragments of antibodies such as Fab fragments, RNA molecules or polypeptides. According to the present invention binding agents may be used isolated or in combination. By means of combination it is possible to achieve a higher degree of sensitivity.

The antibodies useful for the methods according to the present invention may comprise further binding sites for either therapeutic agents or other polypeptides or may be coupled to said therapeutic agents or polypeptides. Therapeutic agents may comprise drugs, toxins, radio-nuclides and derivatives thereof. The agents may be coupled to the binding agents either directly or indirectly for example by a linker or carrier group. The linker group may for example function in order to enable the coupling reaction between binding agent and therapeutic or other agent or the linker may act as a spacer between the distinct parts of the fusion molecule. The linker may also be cleavable under certain circumstances, so as to release the bound agent under said conditions. The therapeutic agents may be covalently coupled to carrier groups directly or via a linker group. The agent may also be non-covalently coupled to the carrier. Carriers that can be used according to the present invention are for example albumins, polypeptides, polysaccharides or liposomes.

The antibody used according to the present invention may be coupled to one or more agents. The multiple agents coupled to one antibody may be all of the same species or may be several different agents bound to one antibody.

Another aspect of the present invention is a pharmaceutical compositon for use in the treatment of disorders associated with abnormal cell proliferation. The polypeptides, polynucleotides and binding agents (esp. antibodies) according to the present invention may be incorporated into pharmaceutical or immunogenic compositions.

The pharmaceutical compositions may be administered by any suitable way known to those of skill in the art. The administration may for example comprise injection, such as e.g., intracutaneous, intramuscular, intravenous or subcutaneous injection, intranasal administration for example by aspiration or oral administration. A suitable dosage to ensure the pharmaceutical benefit of the treatment should be chosen according the parameters, such as age, sex, body weight etc. of the patient, known to those of skill in the art.

The pharmaceutical compositions comprise said compounds and a physiologically acceptable carrier. The type of carrier to be employed in the pharmaceutical compositions of this invention, will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g. polylactic glycolide) may also be employed as carriers for the pharmaceutical compositions of this invention. Suitable biodegradable microspheres are disclosed, for example, in U.S. Pat. Nos. 4,897,268 and 5,075,109.

A pharmaceutical composition or vaccine may for example contain DNA, that codes for one or more polypeptides according to the present invention. The DNA may be administered in a way that allows the polypeptides to be generated in situ. Suitable expression systems are known to those skilled in the art. In another embodiment of the invention the nucleic acids may be for example anti-sense constructs. Pharmaceutical compositions may also comprise nucleic acid molecules expressible in a mammalian or human host system comprising a viral or other expression system for example an adenoviral vector system.

The nucleic acid may also be administered as a naked nucleic acid. In this case appropriate physical delivery systems, which enhance the uptake of nucleic acid may be employed, such as coating the nucleic acid onto biodegradable beads, which are efficiently transported into the cells. Administration of naked nucleic acids may for example be useful for the purpose of transient expression within a host or host cell.

Alternatively the pharmaceutical compositions may comprise one or more polypeptides. The polypeptides incorporated into pharmaceutical compositions may be the inventive colorectal lesion associated polypeptide in combination with one or more other known polypeptides such as for example enzymes, antibodies, regulatory factors, such as e.g. cyclins, cyclin-dependent kinases or CKls, or toxins.

Polypeptides of the present invention or fragments thereof, that comprise an immunogenic portion of a inventive colorectal lesion associated protein, may be used in immunogenic compositions, wherein the polypeptide e.g. stimulates the patient's own immune response to tumor cells. A patient may be afflicted with disease, or may be free of detectable disease. Accordingly, the compounds disclosed herein may be used to treat cancer or to inhibit the development of cancer. The compounds may be administered either prior to or following a conventional treatment of tumors such as surgical removal of primary tumors, treatment by administration of radiotherapy, conventional chemotherapeutic methods or any other mode of treatment of the respective cancer or its precursors.

Immunogenic compositions such as vaccines may comprise one or more polypeptides and a non-specific immune-response enhancer, wherein the non-specific immune response enhancer is capable of eliciting or enhancing an immune response to an exogenous antigen. Any suitable immune-response enhancer may be employed in the vaccines of this invention. For example, an adjuvant may be included. Most adjuvants contain a substance designed to protect the antigen from rapid catabolism, such as aluminium hydroxide or mineral oil, and a non-specific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

Pharmaceutical compositions and vaccines may also contain other epitopes of tumor antigens, either incorporated into a fusion protein as described above (i.e., a single polypeptide that contains multiple epitopes) or present within a separate polypeptide.

A vital aspect of the present invention is the diagnosis of colorectal lesions. The detection of diseases may be carried out for example by detecting the level of expression of one or more molecular markers in biological samples. Molecular markers as used according to the present invention are molecules associated with colorectal lesions. Such molecules may comprise polynucleotides or polypeptides. Polypeptides may comprise the inventive colorectal lesion associated polypeptides or fragments thereof or proteins or fragments thereof interacting with the inventive colorectal lesion associated polypeptides. Preferred nucleic acids for use as molecular markers may comprise nucleic acids coding for the inventive colorectal lesion associated polypeptides or for proteins interacting with the inventive colorectal lesion associated polypeptides, such as for example DNA, cDNA or RNA. In one preferred embodiment of the invention the polynucleotides or polypeptides disclosed to be useful for the diagnostic methods of this invention are the molecular markers to be detected.

Colorectal lesions according to the present invention comprise conditions of the colorectal tract characterized by abnormal growth properties of cells or tissues compared to the growth properties of normal control cells or tissues. The growth of the cells or tissues may be for example abnormally accelerated, decelerated or may be regulated abnormally. Abnormal regulation as used above may comprise any form of presence or absence of non wild-type responses of the cells or tissues to naturally occurring growth regulating influences. The abnormalities in growth of the cells or tissues may be for example neoplastic or hyperplastic. In one preferred embodiment of the invention the colorectal lesions are cancers or precancerous conditions of the colon.

In one preferred embodiment the colorectal lesions are tumors. In another preferred embodiment of the invention the tumors are cancers or precancerours conditions of the colorectal tract.

A sample according to the method of the present invention is any sample, that may contain cells, tissues or body liquids. Furthermore any sample potentially containing the marker molecules to be detected may be a sample according to the present invention. Such samples are e.g. blood, plasma, serum, secretions, such as gastrointestinal secretions or other glandular secretions, smears, washes, stool, bile, cell- and tissue-samples or biopsies.

Biopsies as used in the context of the present invention may comprise e.g. resection samples of tumors, tissue samples prepared by endoscopic means or needle biopsies of organs. Furthermore any sample potentially containing the marker molecules to be detected may be a sample according to the present invention.

The method for detection of the level of the polynucleotides or polypetides according to the present invention is any method, which is suited to detect very small amounts of specific biologically active molecules in samples. The detection reaction according to the present invention may be for example a detection either on the level of nucleic acids or on the level of polypeptides. The detection may either be a detection of the level of polypeptides or nucleic acids in cells in total or in cell lysats or a detection of the level of polypeptides or nucleic acids in distinct subcellular regions. The methods for determining the subcellular distribution of compounds are known to those of skill in the art. In another embodiment of the present invention the detection method may comprise the detection of methylation of nucleic acid molecules in samples.

Applicable formats for the detection reaction according to the present invention may be blotting techniques, such as Western-Blot, Southern-blot, Northern-blot. The blotting techniques are known to those of ordinary skill in the art and may be performed for example as electro-blots, semidry-blots, vacuum-blots or dot-blots. Furthermore immunological methods for detection of molecules may be applied, such as for example immunoprecipitation or immunological assays, such as ELISA, RIA, lateral flow assays etc..

Methods for detection of methylation of nucleic acids are known to those of skill in the art and may comprise for example methods employing chemical pre-treatment of nucleic acids with e.g. sodium bisulphite, permanganate or hydrazine, and subsequent detection of the modification by means of specific restriction endonucleases or by means of specific probes e.g. in the course of an amplification reaction. The detection of methylation may furthermore be performed using methylation specific restriction endonucleases.

In one preferred embodiment of the invention the detection of the level of marker molecules is carried out by detection of the level of nucleic acids coding for the marker molecules or fragments thereof present in the sample. The means for detection of nucleic acid molecules are known to those skilled in the art. The procedure for the detection of nucleic acids can for example be carried out by a binding reaction of the molecule to be detected to complementary nucleic acid probes, proteins with binding specificity for the nucleic acids or any other entities specifically recognizing and binding to said nucleic acids. This method can be performed as well in vitro as directly in-situ for example in the course of a detecting staining reaction. Another way of detecting the marker molecules in a sample on the level of nucleic acids performed in the method according to the present invention is an amplification reaction of nucleic acids, which can be carried out in a semi-quantitative or quantitative manner such as for example PCR, LCR or NASBA. In a preferred embodiment of the present invention real time RT PCR may be used to quantify the level of marker RNA in samples of colorectal lesions.

In another preferred embodiment of the invention the detection of the level of marker molecules is carried out by determining the level of expression of a protein. The determination of the marker molecules on the protein level may for example be carried out in a reaction comprising a binding agent specific for the detection of the marker molecules. These binding agents may comprise for example antibodies and antigen-binding fragments, bifunctional hybrid antibodies, peptidomimetics containing minimal antigen-binding epitopes etc. The binding agents may be used in many different detection techniques for example in western-blot, ELISA, lateral flow assay, latex-agglutination, immunochromatographic strips or immuno-precipitation. Generally, binding agent based detection may be carried out as well in vitro as directly in situ for example in the course of an immuno-cytochemical staining reaction. Any other method suitable for determining the amount of particular polypeptides in solutions of biological samples can be used according to the present invention.

In a further embodiment of the present invention the detection of a series of marker molecules is carried out on the level of polypeptides and simultaneously the detection of a further series of marker molecules and/or of all or some of the same marker molecules is carried out on the level of nucleic acids.

In one preferred embodiment of the invention the expression level of markers is significantly elevated compared to a non tumorous test sample. In this case the marker is overexpressed in the sample. In another preferred embodiment of the present invention the level of the marker is lowered compared to a non tumorous test sample. In a third embodiment there is no detectable expression of the marker at all in the test sample unlike in a control sample. In yet another embodiment there is detectable level of non wild-type marker molecules. Non wild-type marker molecules may comprise any marker molecules that deviate in sequence or structure from the structure or sequence, that is functional in wild type tissue not affected by a cell proliferative disease. Wild type sequences or structures are the sequences or structures predominantly present in normal cells or tissues.

The detection of the level of molecular markers according to the present invention may be the detection of the level of single marker molecules in separated reaction mixtures as well as the detection of a combination of markers simultaneously. The combination may comprise any of the molecular markers disclosed herein. Furthermore there are possible combinations of the markers disclosed with other molecular markers known in the art.

The detection may be carried out in solution or using reagents fixed to a solid phase. The detection of one or more molecular markers may be performed in a single reaction mixture or in two or separate reaction mixtures. The detection reactions for several marker molecules may for example be performed simultaneously in multi-well reaction vessels. The markers disclosed herein may be detected using reagents that specifically recognise these molecules. Simultaneously one or more further markers may be detected using reagents, that specifically recognize them. The detection reaction for each single marker may comprise one or more reactions with detecting agents either recognizing the initial marker molecules or preferably recognizing molecules used to recognize other molecules. Such reaction may e.g. comprise the use of primary and secondary and further antibodies. The detection reaction further may comprise a reporter reaction indicating the level of the inventive polypeptides. The reporter reaction may be for example a reaction producing a coloured compound, a bioluminescence reaction, a fluorescence reaction, generally a radiation emitting reaction etc..

The reporter reaction may be for example a reaction producing a coloured compound. In a preferred embodiment of the present invention the reporter substances correlated to the particular markers develop different colours. In another embodiment the reporter reactions may produce fluorescent dyes with differing wavelength characteristics. In a further embodiment of the present invention the reporter reaction may comprise light emitting reactions with different wavelength characteristics for the reporter substances specific for either marker to be detected. In another embodiment of the present invention the reporter reaction may comprise the emission of radioactive irradiation and additional methods for visualizing or quantifying the irradiation. In a preferred embodiment, the different marker molecules may be recognized by agents, that bear radio-nuclides emitting irradiation with different energetic properties, so that the signals referring to marker molecules could be distinguished.

In one preferred embodiment the detection of tissues expressing marker gene products is carried out in form of molecular imaging procedures. The respective procedures are known to those of ordinary skill in the art. Imaging methods for use in the context of the present invention may for example comprise MRI, SPECT, PET and other methods suitable for in vivo imaging.

In one embodiment the method may be based on the enzymatic conversion of inert or labelled compounds to molecules detectable in the course of molecular imaging methods by the marker molecules. In another embodiment the molecular imaging method may be based on the use of compounds carrying a suitable label for in vivo molecular imaging, such as radio isotopes, metal ions etc., specifically binding to marker molecules in vivo.

In a preferred embodiment of the invention these compounds are non-toxic compounds and may be eliminated from the circulation of organisms, such as humans, in a time span, that allows for performing the detection of label accumulated in tumor tissue overexpressing the respective marker gene. In another preferred embodiment of the invention compounds are used for molecular imaging, for which clearance from the circulation is not relevant for performing the molecular imaging reaction. This may be for example due to low background produced by the circulating molecules etc. The compounds for use in molecular imaging methods are administered in pharmaceutical acceptable form in compositions that may additionally comprise any other suitable substances, such as e.g. other diagnostically useful substances, therapeutically useful substances, carrier substances or the like.

The marker molecules disclosed according to the present invention may be used for diagnosis, monitoring of the disease course and prognosis in colorectal lesions.

Diagnosis of colorectal lesions may for example comprise the detection of cells or tissues affected by abnormal growth. In one preferred embodiment diagnosis means the primary detection of a disease in an organism or sample. According to the present invention the method for diagnosis of the colorectal lesion may be applied in routine screening tests for preventive aspects in order to detect said disease at an early stage of the onset of the disorder. In another preferred embodiment the diagnostic method may be used to determine the minimal residual disease of a tumor after primary therapy. In this respect the method of the invention may be applied to determine cells in body samples displaying abnormal expression of marker molecules according to the present invention, characteristic for colorectal lesions. Thus a spread of affected cells may be detected in body liquids. The determination of disseminated tumor cells according to the present invention may be performed before, during or even after primary treatment of colorectal lesions and may for example be performed for purposes of staging. In this context the detection of the compounds disclosed herein may comprise the detection of spread tumor cell to lymph nodes.

In one embodiment of the invention the methods disclosed herein may be used for the detection and identification of metastases. The method may be applied either for detection of metastases in body tissues or organs by the detection methods described herein, or the metastases may be diagnoses with respect to prognosis and prediction of disease course.

Monitoring of the disease course may comprise determining the levels of a set of marker molecules at different time points, comparing the levels at the different time points and assessing a diagnosis about the progression of the disease over the covered period of time. Thus monitoring may enable for assessment of prognosis and/or for design of an adequate therapy for a particular patient.

Prognosis of the disease course of a colorectal lesion according to the present invention may comprise determining the level of expression of one or more marker molecules, comparing the levels with data from subsequent studies in a database and prognosticating the disease course from said comparison. In a preferred embodiment the method may comprise the detection of the levels of a set of marker molecules, the distinct levels of which may characterize distinct stages in the course of the disease. In a further embodiment of the invention the combination of the levels of a combination of markers may be an indicator for the prognosis of the further disease course and may build the basis for design of an adequate therapy.

The present invention further provides kits for use in e.g. research or diagnostic methods. Such kits may contain two or more components for performing a scientific or diagnostic assay. Components may be compounds, reagents, containers and/or equipment. One component may be an antibody or fragment thereof that specifically binds to a polypeptide associated with colorectal lesions. Additionally the kit may contain reagents, buffers or others known in the art as necessary for performing the diagnostic assay. Alternatively the research kit or diagnostic kit may contain nucleotide probes or primers for the detection of DNA or RNA. Such a kit could contain appropriate additional reagents and buffers known in the art.

A kit according to present invention comprises:
a) reagents for the detection of the molecular marker molecules
b) the reagents and buffers commonly used for carrying out the detection reaction, such as buffers, detection-markers, carrier substances and others
d) a marker sample for carrying out a positive control reaction.

The reagent for the detection of the marker includes any agent capable of binding to the marker molecule. Such reagents may include proteins, polypeptides, peptidomimetics, nucleic acids, glycoproteins, proteoglycans, polysaccharids, anticalins or lipids.

The sample for carrying out a positive control may comprise for example nucleic acids in applicable form, such as solution or salt, peptides in applicable form, tissue section samples or positive cells expressing the molecules associated with colorectal lesions.

In a preferred embodiment of the invention the detection of the marker molecules is carried out on the level of polypeptides. In this embodiment the binding agents may be for example antibodies specific for the marker molecules or fragments thereof.

In an other embodiment of the test kit the detection of the marker molecule is carried out on the nucleic acid level. In this embodiment of the invention the reagents for the detection may be for example nucleic acid probes or primers complementary to said marker molecule nucleic acids.

Another aspect of the present invention is to provide a method for therapy and/or vaccination. According to the present invention a therapy of cell proliferative disorders can be carried out using the inventive colorectal lesion associated polypeptides and/or polynucleotides. The therapy may be for example immunotherapy or somatic gene therapy.

The inventive colorectal lesion associated polypeptides and/or polynucleotides may according to the present invention be used for vaccination against cell proliferative disorders. Vaccination according to the present invention may comprise administering an immunogenic compound to an individual for the purpose of stimulating an immune response directed against said immunogenic compound and thus immunizing said individual against said immunogenic compound. Stimulating an immune response may comprise inducing the production of antibodies against said compound as well as stimulating cytotoxic T-cells. For the purpose of vaccination the polypeptides, nucleic acids and binding agents according to the present invention may be administered in a physiological acceptable form. The composition to be administered to individuals may comprise one or more antigenic components, physiologically acceptable carrier substances or buffer solutions, immunostimulants and/or adjuvants. Adjuvants may comprise for example Freund's incomplete adjuvant or Freund's complete adjuvant or other adjuvants known to those of skill in the art.

The composition may be administered in any applicable way such as e.g. intravenous, subcutaneous, intramuscular etc.. The dosage of the composition depends on the particular case and purpose of the vaccination. It has to be adapted to parameters by the individual treated such as age, weight, sex etc.. Furthermore the type of the immune response to be elicited has to be taken into account. In general it may be preferable if an individual receives 100 µg - 1 g of a polypeptide according to the present invention or 10⁶ - 10¹² MOI of a recombinant nucleic acid, containing a nucleic acid according to the present invention in a form that may be expressed in situ.

Individuals for the purpose of vaccination may be any organisms containing the inventive colorectal lesion associated polypeptides and/or polynucleotides and being able to get affected by cell proliferative disorders.

Vaccination of individuals may be favourable e.g. in the case of altered, non wild-type sequences or structure of marker molecules associated with cell proliferative disorders.

Polypeptides disclosed herein may also be employed in adoptive immunotherapy for the treatment of cancer. Adoptive immunotherapy may be broadly classified into either active or passive immunotherapy. In active immunotherapy, treatment relies on the in vivo stimulation of the endogenous host immune system to react against tumors with the administration of immune response-modifying agents (for example, tumor vaccines, bacterial adjuvants, and/or cytokines).

In passive immunotherapy, treatment involves the delivery of biologic reagents with established tumor-immune reactivity (such as effector cells or antibodies) that can directly or indirectly mediate antitumor effects and does not necessarily depend on an intact host immune system. Examples of effector cells include T lymphocytes (for example, CD8+ cytotoxic T-lymphocyte, CD4+ T-helper, tumor-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Pat. No. 4,918,164), for passive immunotherapy.

The predominant method of procuring adequate numbers of T-cells for adoptive immunotherapy is to grow immune T-cells in vitro. Culture conditions for expanding single antigen-specific T-cells to several billion in number with retention of antigen recognition in vivo are well known in the art. These in vitro culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage or B-cells, may be pulsed with immunoreactive polypeptides or transfected with a nucleic acid sequence(s), using standard techniques well known in the art. For example, antigen presenting cells may be transfected with a nucleic acid sequence, wherein said sequence contains a promoter region appropriate for increasing expression, and can be expressed as part of a recombinant virus or other expression system. For cultured T-cells to be effective in therapy, the cultured T-cells must be able to grow and distribute widely and to survive long term in vivo. Studies have demonstrated that cultured T-cells can be induced to grow in vivo and to survive long term in substantial numbers by repeated stimulation with antigen supplemented with IL-2 (see, for example, Cheever, M., et al, "Therapy With Cultured T Cells: Principles Revisited," Immunological Reviews, 157:177, 1997).

The polypeptides disclosed herein may also be employed to generate and/or isolate tumor-reactive T-cells, which can then be administered to the patient. In one technique, antigen-specific T-cell lines may be generated by in vivo immunization with short peptides corresponding to immunogenic portions of the disclosed polypeptides. The resulting antigen specific CD8+ CTL clones may be isolated from the patient, expanded using standard tissue culture techniques, and returned to the patient.

Alternatively, peptides corresponding to immunogenic portions of the polypeptides of the invention may be employed to generate tumor reactive T-cell subsets by selective in vitro stimulation and expansion of autologous T-cells to provide antigen-specific T-cells which may be subsequently transferred to the patient as described, for example, by Chang et al. (Crit. Rev. Oncol. Hematol., 22(3), 213, 1996). Cells of the immune system, such as T-cells, may be isolated from the peripheral blood of a patient, using a commercially available cell separation system, such as CellPro Incorporated's (Bothell, Wash.) CEPRATE.™. system (see U.S. Pat. No. 5,240,856; U.S. Pat. No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T-cells. The population of tumor antigen-specific T-cells is then expanded using standard techniques and the cells are administered back to the patient.

In another embodiment, T-cell and/or antibody receptors specific for the polypeptides can be cloned, expanded, and transferred into other vectors or effector cells for use in adoptive immunotherapy.

In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T-cells to provide antigen-specific T-cells, which may, in turn, be administered to a patient. The use of peptide-pulsed dendritic cells to generate antigen-specific T-cells and the subsequent use of such antigen-specific T-cells to eradicate tumors in a murine model has been demonstrated by Cheever et al, Immunological Reviews, 157:177,1997.

Additionally, vectors expressing the disclosed nucleic acids may be introduced into stem cells taken from the patient and clonally propagated in vitro for autologous transplant back into the same patient.

Monoclonal antibodies of the present invention may also be used as therapeutic compounds in order to diminish or eliminate tumors. The antibodies may be used on their own (for instance, to inhibit metastases) or coupled to one or more therapeutic agents. Suitable agents in this regard include radio nuclides, differentiation inducers, drugs, toxins, and derivatives thereof. Preferred radio nuclides include 90Y, 123l, 125l, 131l, 186Re, 188Re, 211At, and 212Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

In one embodiment of the invention the therapy of colorectal lesions may comprise the administration of antisense constructs or ribozymes. The methods for administration of ribozymes or antisense constructs are known to those of skill in the art. The administration may take place as administration of naked nucleic acids or as administration of nucleic acids that are suited for expression of the relevant active products in situ.

In another embodiment of the invention the treatment of disorders may comprise the administration of binding agents directed against the inventive colorectal lesion associated molecules. These binding agents may for example be coupled to other compounds such as toxins, enzymes, radioisotopes etc.

The present invention provides methods for detection and treatment of colorectal lesions, such as e.g. colon carcinomas or adenomas of the colon. In one aspect the present invention provides a method for the detection of disorders characterized by abnormal cell proliferation, such as e.g. cancers based on the determination of the presence or absence and/or the level of expression of the genes associated with colorectal lesions in biological samples. In a second aspect the present invention provides a method for treatment of colorectal lesions, such as e.g. cancers using the inventive colorectal lesion associated gene products as therapeutically active agents. The invention also provides for therapeutic methods based on the modulation of the activity of the inventive colorectal lesion associated polypeptides. It is one aspect of the invention to provide a method for rational tumor management based on the detection of the colorectal lesion associated gene products in patient samples and the tailoring of a therapy correlated to the detected expression of said gene products. Furthermore the present invention provides for a research or diagnostic test kit for performing the reactions involved in the detection of the presence or absence and/or the level of expression of the inventive colorectal lesion associated gene. Finally the present invention relates to pharmaceutical compositions applicable in the treatment of colorectal lesions according to the present invention.

### Brief description of the drawings:

- Figure 1:: **Cloned Rsal restriction fragment of the PEP19 (PCP4) gene (Acc. No. U52969)** that was spotted in duplicates on nylon membranes and hybridized with the normal colon subtracted probe (N) and the colon carcinoma subtracted probe (T), respectively.
- Figure 2:: **Cloned Rsal restriction fragment of the ENA78 gene (Acc. No. Af349466)** that was spotted in duplicates on nylon membranes and hybridized with the normal colon subtracted probe (N) and the colon carcinoma subtracted probe (T), respectively.
- Figure 3:: **Cloned Rsal restriction fragment of the elF-1AY gene (Acc. No. Af000987)** that was spotted in duplicates on nylon membranes and hybridized with the normal colon subtracted probe (N) and the colon carcinoma subtracted probe (T), respectively.
- Figure 4:: **Table displaying the genes associated with colorectal lesions.** The genes given in this table are differentially expressed in tissue of colorectal tumors; The genes are overexpressed in tumor tissue and not in corresponding normal tissue. (see example 1 and 2) These genes may be used for detection and treatment of colorectal disorders according to the present invention.
- Figure 5:: **Results of the RT-PCR experiment.** (for experimental details see example 2) The figure shows the expression of the FN14 gene in samples of colon carcinomas related to the expression in corresponding normal colon tissue. The y-axis shows the rate of overexpression related to the control expression in normal control tissue

The following examples are given for the purpose of illustration only and are not intended to limit the scope of the invention disclosed herein.

### Example 1: Identification of differential expression of genes in human colon tissue versus human colon tumor tissue

### (i) Isolation of total RNA:

Total RNA was extracted from human colon tissue by using the RNeasy kit (Qiagen) according to the manufacturer's protocol. The quality of the prepared RNA was determined by agarose gel electrophoresis according to the standard procedures (Sambrook J, et al., 1989). The mRNA fraction was amplified using the SMART™ PCR cDNA synthesis kit (Clontech).

### (ii) Identification of differentially expressed genes may for example be performed by suppressive subtractive hybridisation method as follows:

This technique compares two populations of mRNA and provides clones of genes that are expressed in one population but not in the other. The applied technique was described in detail in Diatchenko et al., 1996. In the present invention, mRNA populations from human colon cancer tissue and normal colon tissue were compared. Specifically, mRNA of the normal colon biopsy was subtracted from mRNA of the colon cancer tissue. The necessary reagents were taken from the PCR-Select™ cDNA subtraction kit (Clontech) and all steps were performed as described in the manufacturer's protocol.

In detail, 2µg SMART™-amplified double-stranded cDNA was used. After Rsal-digestion and adaptor ligation hybridization of tester and driver were performed for 8 h (first hybridization) and 15 h (second hybridization) at 68°C. Two PCR steps were performed to amplify differentially expressed genes (first PCR: 27 cycles of 94°C 30 s, 66°C 30 s and 72°C 1.5 min; nested PCR: 12 cycles of 94°C 30 s, 66°C 30 s and 72°C 1.5 min) using adaptor specific primers (included in the subtraction kit) and 50x Advantage Polymerase Mix (Clontech). Efficiencies of Rsal-digestions, adaptor ligations and subtractive hybridizations were checked as recommended in the kit.

Subtracted cDNAs were inserted into the pCR2.1 vector and transformed into INVαF' cells (Invitrogen).

To isolate individual cDNAs of the subtracted library single bacterial transformants were incubated in 100 µl LB amp (50 µg/ml) at 37°C for at least 4 hours. Inserts were PCR amplified (95°C 30 sec, 68°C 3 min for 30 cycles) in 20 µl containing 10 mM Tris-HCI pH 9.0, 1.5 mM MgCI2, 50 mM KCI, 200 µM dNTP, 0.5 µM adaptor specific primers (included in the subtraction kit), 1.5 Units Taq polymerase (Pharmacia Biotech), and 1µl of bacterial culture.

1.5 µl of a mixture containing 3 µl PCR amplified inserts and 2 µl 0.3 N NaOH/15% Ficoll were spotted onto a positively charged nylon membrane (Roche). In this way, hundreds of spots were arrayed on duplicate filters for subsequent hybridization. The differential screening step consists of hybridizations of the subtracted library with itself to minimize background (Wang et al., 1991). The probes were made of the nested PCR product of the subtraction following the instructions of the Clontech subtraction kit. Labeling with Digoxigenin was performed with the DIG DNA Labeling Kit (Roche). Hybridizations were carried out overnight in DIG Easy HYB (Roche) at 43°C. The filters were washed twice in 2 x SSC / 0.5% SDS at 68°C for 15 min and twice in 0,1 x SSC / 0.5% SDS at 68°C for 15 min, and subjected to detection using anti-DIG-AP conjugates and CDP-Star™ as chemiluminescent substrate according to the instructions of the DIG DNA Detection Kit (Roche). Blots were exposed to Kodak Biomax MR chemiluminescent film at room temperature for several minutes.

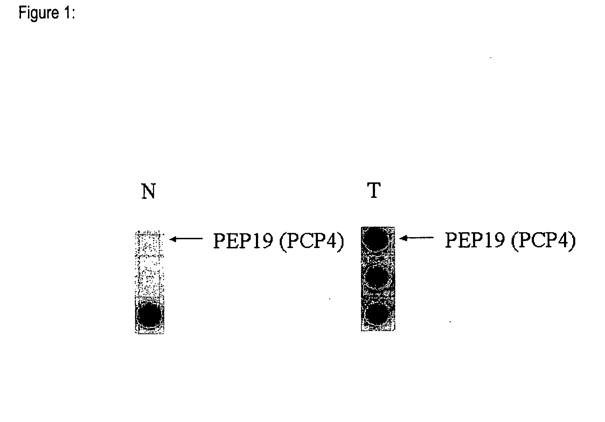

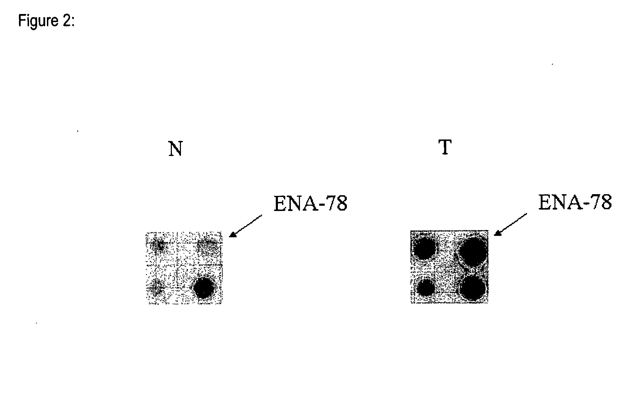

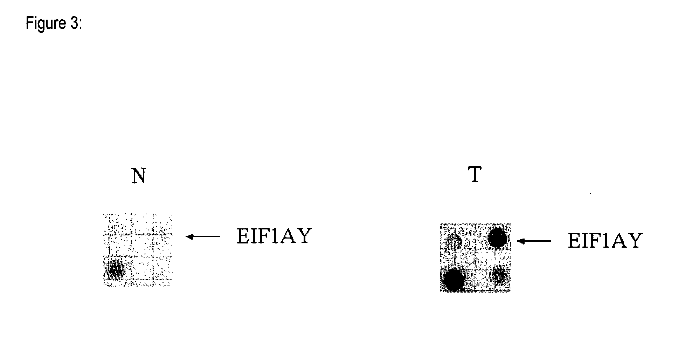

The genes given in figure 4 have been identified to be differentially expressed in colorectal lesions compared to normal tissue. Examples of the differential screening of the subtracted libraries are shown in figures 1-3.

### Example 2 : Detection of the level of expression of genes associated with colorectal disorders by RT-PCR

Samples of adenocarcinomas and adenomas of the colon are used to determine the level of mRNA of the genes given in the table displayed in Figure 4 using semi-quantitative RT PCR. 15 tumor biopsies and samples of 50 adenomas are used in this study.

Tumors and adenomas are collected, snap frozen, and stored at -80°C. They are verified to be composed predominantly of neoplastic cells by histopathological analysis. mRNA is isolated from tumors and patient-matched normal tissue using Qiagen reagents (Qiagen, Hilden, Germany), and single-stranded cDNA is synthesized using Superscript II (Life Technologies, Inc.). Quantitative PCR is performed using the 7700 Sequence Detector (TaqmanTM) and the SYBR Green PCR Master-Mix, as described in the manufacturers manual (Applied Biosystems, Foster City, CA).

PCR reactions are performed in 25 µl volumes with a final concentration of 300 nmol for each primer, with 95°C for 15 sec and 60°C for 60 sec, for 40 cycles.

The specificity of the PCR products is verified by gel electrophoresis (data not shown).

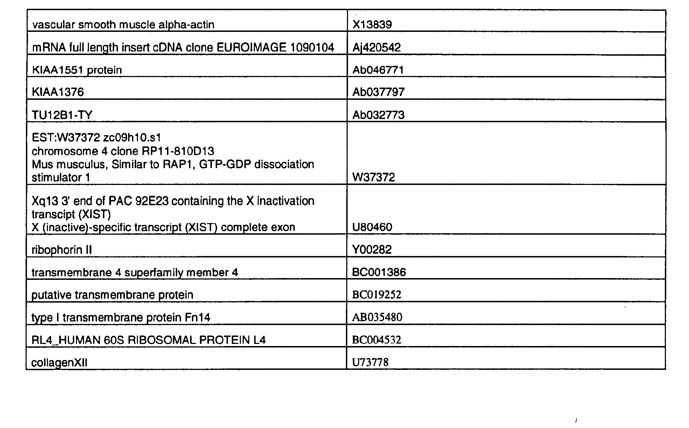

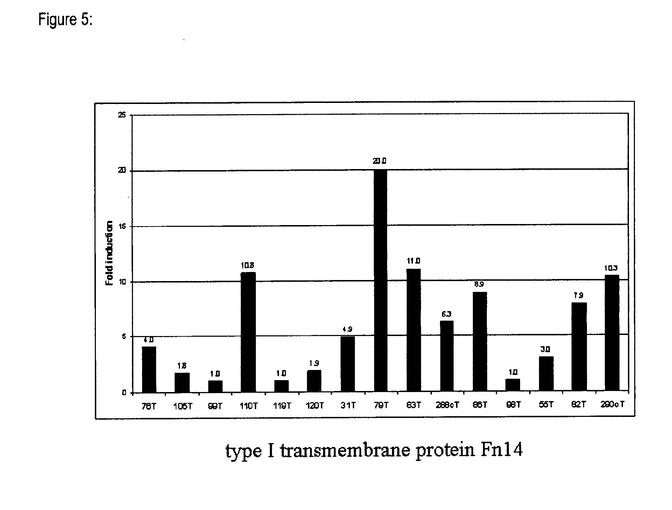

The results show, that the genes identifiable by the method described in example 1 are overexpressed in tumor tissue in comparison to normal colon mucosa tissue. Figure 5 shows the results of RT-PCR experiments for the FN14 in samples of colon carcinomas. The results obtained for the other genes presented in the table given in Figure 4 are comparable to these data. Also the results obtained from the analysis of adenomas of the colon show overexpression of the tested genes listed in Figure 4.

The example illustrates, that the genes identified herein are suitable for the detection of colorectal lesions by determining the expression level of the respective genes given in figure 4. A comparison of the expression level of the particular marker molecules in test tissue to the expression level in normal control tissue reveals whether the test tissue is affected by a colorectal lesion or not.

## Claims

1. An isolated nucleic acid molecule associated with colorectal lesions for use in the detection and therapy of colorectal lesions being selected from a group consisting of
a. a nucleic acid sequence of a gene given in Figure 4;
b. a nucleic acid molecule encoding a polypeptide the sequence of which shows at least 65% identity to the amino acid sequence of the polypeptide encoded by a nucleic acid molecule specified in (a);
c. a nucleic acid molecule the sequence of which differs from the sequence of a nucleic acid molecule of (a) to (b) due to the degeneracy of the genetic code;
d. a nucleic acid molecule, which represents a fragment or an allelic variant of a nucleic acid molecule of (a) to (c); and
e. a nucleic acid, which encodes a fragment or a variant of the amino acid sequence encoded by a gene given in Figure 4, which has an increased or decreased biological activity compared to the wild type amino acid sequence.

2. An isolated polypeptide molecule associated with colorectal lesions for use in the detection and therapy of colorectal lesions being selected from a group consisting of
(a) a polypeptide, which is encoded by a nucleic acid molecule of claim 1;
(b) a polypeptide, that is recognized by a binding agent, that has been raised against and is specifically binding the polypeptide of (a);
(c) a fragment or a variant of the polypeptides of (a)-(b), that is encoded by a nucleic acid sequence, that hybridizes to a nucleic acid according to claim 1 under stringent conditions; and
(d) a fragment or variant of the polypeptides of (a)-(c), which has an increased or decreased biological activity compared to the wild polypeptides associated with colorectal lesions.

3. A fusion polypeptide or chimeric nucleic acid for use in the detection or therapy of colorectal lesions comprising a polypeptide or nucleic acid or fragments thereof according to claims 1 or 2.

4. A nucleic acid being DNA or RNA that is reverse complementary or complementary to a nucleic acid of claim 1 for use in diagnosis or therapy of colorectal lesions.

5. A binding agent specifically recognizing and binding to a molecule of any one of the claims 1-5 for use in detection or therapy of colorectal lesions.

6. A nucleic acid or a polypeptide according to any one of the claims 1-4 or a binding agent according to claim 5, which is detectably labelled.

7. A nucleic acid, a polypeptide or a binding agent according to claim 6, wherein the label is selected from the group consisting of a radioisotope, a bioluminescent compound, a chemiluminescent compound, a fluorescent compound, a metal chelate, a biologically relevant binding structure such as biotin or digoxygenin or an enzyme.

8. Method for diagnosis of colorectal lesions and/or prognosis of disease course comprising
a. obtaining a sample from an individual
b. determining the levels of one or more marker molecules being nucleic acid molecules or polypeptides according to claim 1 or 2
c. comparing the levels of said nucleic acids or polypeptides within said sample to the contents within a corresponding test sample, not affected by the disease being tested,
wherein the diagnosis or prognosis of disease course is predicted from considering a significant change relative to the wild type level of a single marker molecule or of a set of marker molecules as indicative of said colorectal lesion or of the disease course.

9. The method of claim 8, wherein the sample is a liquid containing nucleic acids, polypeptides or fragments thereof, a liquid containing cells or cell debris, a tissue sample, a cell sample or a biopsy.

10. The method of claim 8 or claim 9, wherein the sample is blood, plasma, serum, a secretion, a smear, stool, bile, cell- and tissue-samples or biopsies.

11. The method according to any one of the claims 8-10, wherein the colorectal lesion is a benign and malignant tumor, a carcinoma or a dysplasia.

12. The method according to claim 11, wherein the tumor is cancer of the of the gastrointestinal tract.

13. The method according to claims 8-12, wherein the disease is colon cancer.

14. A method according to any one of the claims 8-13, wherein the detection of the expression of the marker molecules is carried out using at least one probe specifically binding to the marker molecules to be detected.

15. A method according to claim 14, wherein the probe is detectably labelled.

16. The method according to claim 15, wherein the label is selected from the group consisting of a radioisotope, a bioluminescent compound, a chemiluminescent compound, a fluorescent compound, a metal chelate, a biologically relevant binding structure such as biotin or digoxygenin or an enzyme.

17. The method according to claims 14-16 , wherein at least one probe is a binding agent directed against said marker molecules.

18. The method of claim 17 wherein the binding agent is an antibody, a fragment of an antibody, a peptidomimetic comprising an antigen binding epitope or a mini-antibody.

19. The method according to any one of the claims 14-18, wherein the detection comprises an immuno-cytochemical detection procedure.

20. The method according to claim 14-16, wherein at least one probe is a nucleic acid hybridising to a marker nucleic acid is used for the detection of the marker molecules.

21. The method according to any one of the claims 20, wherein the detection reaction comprises a nucleic acid amplification reaction.

22. The method according to claim 21, wherein the amplification reaction is PCR, LCR or NASBA.

23. The method according to any one of the claims 14-22, which is used for in-situ detection.

24. A method according to any one of the claims 8-23, which is used in the course of an in vivo or in vitro molecular imaging method.

25. A method according to any one of the claims 8-24, which is carried out in the course of early diagnosis of disorders, of minimal residual disease diagnosis or of preventive screening tests.

26. A test-kit for carrying out the method according to any one of the claims 8-25, comprising at least
a. reagents for the detection of polynucleotides and/or polypeptides
b. the reagents and buffers commonly used for carrying out the detection reaction, such as buffers, detection-markers, carrier substances and others

27. A test-kit according to claim 26, wherein the reagent for detection of the polynucleotide and/or polypeptide is an agent specifically binding to said polynucleotide and/or polypeptide.

28. Use of one or more compounds according to any one of the claims 1-8 for production of a medicament for treating colorectal lesions.

29. Use according to claim 28, wherein the colorectal lesion is a benign and malignant tumor, a carcinoma or a dysplasia.

30. Use according to any one of the claims 28-29, wherein the medicament is used in immunotherapy or in vaccination therapy.

31. A pharmaceutical composition comprising one or more compounds according to any one of the claims 1-8 useful for treatment of colorectal lesions.
